(19) Europäisches Patentamt
European Patent Office
Office européen des brevets

(11) Veröffentlichungsnummer: **0 095 591**
**B1**

# (12) EUROPÄISCHE PATENTSCHRIFT

(45) Veröffentlichungstag der Patentschrift: **08.01.86**

(51) Int. Cl.⁴: **B 01 F 17/22**, A 23 J 7/00, B 01 F 1/00

(21) Anmeldenummer: **83104406.0**

(22) Anmeldetag: **05.05.83**

(54) **Phospholipidlösungen.**

(30) Priorität: **13.05.82 DE 3218027**

(43) Veröffentlichungstag der Anmeldung: **07.12.83 Patentblatt 83/49**

(45) Bekanntmachung des Hinweises auf die Patenterteilung: **08.01.86 Patentblatt 86/2**

(84) Benannte Vertragsstaaten: **AT BE CH DE FR GB IT LI LU NL SE**

(56) Entgegenhaltungen:
**AT - B - 336 550**
**DD - B - 137 784**
**DE - A - 2 512 639**
**DE - B - 2 023 427**
**US - A - 2 660 567**

**Chemical Abstracts, Band 77, Nr. 20, 13. November 1972, Columbus, Ohio, USA, P.A. DEMCHENKO et al., "Critical micelle concentration (CMC) of sodium alkylphosphonates as affected by additions of polar substances", Seite 308, spalte 1, abstract nr. 131083s**

(73) Patentinhaber: **A. Nattermann & Cie. GmbH, Nattermannallee 1, D-5000 Köln 30 (DE)**

(72) Erfinder: **Biedermann, Jürgen, Dr., Kirschenweg 14, D-5024 Pulheim-Stommeln (DE)**
Erfinder: **Betzing, Hans, Dr., Heidestock 7, D-5014 Kerpen-Horrem (DE)**

(74) Vertreter: **Redies, Bernd, Dr. rer. nat., COHAUSZ & FLORACK Patentanwaltsbüro Schumannstrasse 97 Postfach 14 01 47, D-4000 Düsseldorf 1 (DE)**

**EP 0 095 591 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind wässerige Phospholipidlösungen sowie ihre Herstellung gemäss den Ansprüchen 1-11.

Phospholipide sind natürliche oder synthetische Produkte. Sie finden in der Lebensmittel-, Pharmazeutischen- sowie Technischen Industrie vielfältige Anwendung (B.F. Szukaj in World Soybean Res. Conf. II 1979 – Westview Press, S. 681-91).

Phospholipide sind in der Regel plastische, technisch schwer zu verarbeitende Massen, welche in organischen Lösungsmitteln, wie z.B. Hexan oder in Mineralöl u.dgl. löslich sind, jedoch unlöslich in Wasser. Bei vielen Anwendungen sind jedoch organische Lösungsmittel unerwünscht. Wegen der Unlöslichkeit in Wasser müssen dann Emulsionen hergestellt werden, die oft nicht stabil genug sind oder nur beschränkt einsetzbar. Es ist daher wünschenswert, klare wässerige Lösungen von Phospholipiden zu haben, die leicht bei der Anwendung in die verschiedensten Produkte eingearbeitet werden können.

Es wurde nun überraschend gefunden, dass Hydroxyethylamide der allgemeinen Formel I

$$CH_3-(CH_2)_n-CONHCH_2-CH_2OH \quad I$$

in der n = 3, 4 oder 5 bedeutet, als Lösungsvermittler eingesetzt, klare wässerige Phospholipidlösungen ergeben. Die erhaltenen Lösungen haben einen neutralen pH und sind auch bei längerer Lagerung stabil.

Als Hydroxyethylamide kommen
N-(2-Hydroxyethyl)-valeriansäureamid,
N-(2-Hydroxyethyl)-capronsäureamid oder
N-(2-Hydroxyethyl)-heptansäureamid
in Frage.

Als Phospholipide können alle natürlichen wie synthetischen Phospholipide eingesetzt werden.

Als natürliche Phospholipide kommen insbesondere in Betracht Phosphatidylcholin, Phosphatidylethanolamin, Phosphatidylinosit, Lysophosphatidylcholin, N-Acylphosphatidylethanolamin und Phosphatidsäure sowie Mischungen dieser Phospholipide.

Beispiele für Mischungen von Phospholipiden sind

| | | |
|---|---|---|
| A) | Phosphatidylcholin | 50,1% |
| | Phosphatidylethanolamin | 23,7% |
| | Lysophosphatidylcholin | 1,7% |
| | Phosphatidylinosit | 6,1% |
| | N-Acylphosphatidylethanolamin | 5,2% |
| | Phosphatidsäure | 2,1% |
| | Glycolipide und Triglyceride | 11,2% |
| B) | Phosphatidylcholin | 11,4% |
| | Phosphatidylethanolamin | 27,4% |
| | Lysophosphatidylcholin | 1,3% |
| | Phosphatidylinosit | 27,0% |
| | N-Acylphosphatidylethanolamin | 1,2% |
| | Phosphatidsäure | 15% |
| | Glycolipide und Triglyceride | 16,5% |
| C) | Phosphatidylcholin | 26,2% |
| | Phosphatidylethanolamin | 25,1% |
| | Lysophosphatidylcholin | 1,3% |
| | Phosphatidylinosit | 21,4% |
| | N-Acylphosphatidylethanolamin | 5,3% |
| | Phosphatidsäure | 12,6% |
| | Glycolipide und Triglyceride | 7,8% |

Das überraschende Solubilisierungsvermögen der nichtionogenen Fettsäure –N–(2-hydroxyethyl)–amide für Phospholipide nimmt in der homologen Reihe mit steigender Kettenlänge der Fettsäuren zu: $C_5 < C_6 < C_7$. Die danach zu erwartende Verringerung der zur Solubilisierung notwendigen Menge beim Übergang zur $C_8$-Säure (Octansäure) kann praktisch nicht genutzt werden, da das Hydroxyethylamid der Octansäure wasserunlöslich ist.

Die zur vollständigen Wasserlöslichkeit des Phospholipids notwendige Mindestmenge an Solubilisator ist von der Kettenlänge des verwendeten Hydroxyethylamids abhängig. Während beim N-(2-Hydroxyethyl)-valeriansäureamid 1,5 Teile auf 1 Teil Phospholipid notwendig sind, genügen beim N-(2-Hydroxyethyl)-capronsäureamid schon 1,2 Teile auf 1 Teil Phospholipid, beim N-(2-Hydroxyethyl)-heptansäureamid ist vollständige Wasserlöslichkeit des Phospholipids schon mit 0,75 Teilen zu erreichen.

Die Konzentrationen der Phospholipide in der Lösung sind bei Verwendung dieser pH-neutralen nichtionogenen Solubilisatoren in weiten Grenzen variabel und ermöglichen Lösungen mit einem Gehalt von 1-25% an Phospholipid, vorzugsweise mit einem Gehalt von 5-10% an Phospholipid.

Die für die Herstellung der erfindungsgemässen Phospholipidlösungen notwendigen Hydroxyethylamide wurden nach Vorschrift von G.F. D'Alelio und E. Emmet Reid, J. Am. Soc. 59, 111-112 (1937) erhalten. Die Herstellung der erfindungsgemässen wässerigen Phospholipidlösungen erfolgt in der Weise, dass man zunächst das entsprechende Hydroxyethylamid bei 20° C in ca. einem Zehntel der für die gewünschte Endkonzentration der Phospholipidlösung erforderlichen Wassermenge löst. Anschliessend wird das Phospholipid zugefügt und solange bei 20° C gerührt, bis eine klare Lösung von öliger Konsistenz entstanden ist. Die Restwassermenge wird unter weiterem Rühren langsam zugesetzt bis eine klare Phospholipidlösung erhalten wird, die einen pH-Wert um den Neutralpunkt hat und bei Raumtemperatur stabil ist.

Die Herstellung der erfindungsgemässen Phospholipidlösungen wird durch die folgenden Beispiele näher erläutert.

*Beispiel 1*

7,5 g N-(2-Hydroxyethyl)-valeriansäureamid werden bei 20° C in 10 ml Wasser gelöst. Nach Zugabe von 5 g Phospholipid der Zusammensetzung

| | |
|---|---|
| Phosphatidylcholin | 96% |
| Lysophosphatidylcholin max. | 3% |
| Triglyceride max. | 1% |

wird solange bei 20° C gerührt, bis eine klare Lösung von öliger Konsistenz entstanden ist. 77,5 ml Wasser werden unter weiterem Rühren langsam

zugesetzt, und man erhält eine klare, stabile Phospholipidlösung, die einen pH-Wert um den Neutralpunkt hat.

*Beispiel 2*

12 g N-(2-Hydroxyethyl)-capronsäureamid werden bei 20° C in 10 ml Wasser gelöst. Nach Zugabe von 10 g Phospholipid der Zusammensetzung aus Beispiel 1 wird solange bei 20° C gerührt, bis eine klare Lösung von öliger Konsistenz entstanden ist. 68 ml Wasser werden unter weiterem Rühren langsam zugesetzt, und man erhält eine klare, stabile Phospholipidlösung, die einen pH-Wert um den Neutralpunkt hat.

*Beispiel 3*

15 g N-(2-Hydroxyethyl)-heptansäureamid werden bei 20° C in 10 ml Wasser gelöst. Nach Zugabe von 20 g Phospholipid der Zusammensetzung aus Beispiel 1 wird solange bei 20° C gerührt, bis eine klare Lösung von öliger Konsistenz entstanden ist. 65 ml Wasser werden unter weiterem Rühren langsam zugesetzt, und man erhält eine klare, stabile Phospholipidlösung, die einen pH-Wert um den Neutralpunkt hat.

*Beispiel 4*

4,5 g N-(2-Hydroxyethyl)-heptansäureamid werden bei 20° C in 10 ml Wasser gelöst. Nach Zugabe von 6 g Phospholipid der Zusammensetzung

| | |
|---|---|
| Phosphatidylcholin | 50,1% |
| Phosphatidylethanolamin | 23,7% |
| Lysophosphatidylcholin | 1,7% |
| Phosphatidylinosit | 6,1% |
| N-Acylphosphatidylethanolamin | 5,2% |
| Phosphatidsäure | 2,1% |
| Glycolipide und Triglyceride | 11,2% |

wird solange bei 20° C gerührt, bis eine klare Lösung von öliger Konsistenz entstanden ist. 79,5 ml Wasser werden unter weiterem Rühren langsam zugesetzt, und man erhält eine klare, stabile Phospholipidlösung, die einen pH-Wert um den Neutralpunkt hat.

*Beispiel 5*

12 g N-(2-Hydroxyethyl)-capronsäureamid werden bei 20° C in 10 ml Wasser gelöst. Nach Zugabe von 10 g Phospholipid der Zusammensetzung aus Beispiel 4 wird solange bei 20° C gerührt, bis eine klare Lösung von öliger Konsistenz entstanden ist. 68 ml Wasser werden unter weiterem Rühren langsam zugesetzt, und man erhält eine klare, stabile Phospholipidlösung, die einen pH-Wert um den Neutralpunkt hat.

*Beispiel 6*

12 g N-(2-Hydroxyethyl)-heptansäureamid werden bei 20° C in 10 ml Wasser gelöst. Nach Zugabe von 16 g Phospholipid der Zusammensetzung aus Beispiel 4 wird solange bei 20° C gerührt, bis eine klare Lösung von öliger Konsistenz entstanden ist. 62 ml Wasser werden unter weiterem Rühren langsam zugesetzt, und man erhält eine klare, stabile Phospholipidlösung, die einen pH-Wert um den Neutralpunkt hat.

*Beispiel 7*

1,5 g N-(2-Hydroxyethyl)-heptansäureamid werden bei 20° C in 10 ml Wasser gelöst. Nach Zugabe von 2 g Phospholipid der Zusammensetzung

| | |
|---|---|
| Phosphatidylcholin | 11,4% |
| Phosphatidylethanolamin | 27,4% |
| Lysophosphatidylcholin | 1,3% |
| Phosphatidylinosit | 27,0% |
| N-Acylphosphatidylethanolamin | 1,2% |
| Phosphatidsäure | 15,0% |
| Glycolipide und Triglyceride | 16,5% |

wird solange bei 20° C gerührt, bis eine klare Lösung von öliger Konsistenz entstanden ist. 86,5 ml Wasser werden unter weiterem Rühren langsam zugesetzt, und man erhält eine klare, stabile Phospholipidlösung, die einen pH-Wert um den Neutralpunkt hat.

*Beispiel 8*

9,6 g N-(2-Hydroxyethyl)-capronsäureamid werden bei 20° C in 10 ml Wasser gelöst. Nach Zugabe von 8 g Phospholipid der Zusammensetzung aus Beispiel 7 wird solange bei 20° C gerührt, bis eine klare Lösung von öliger Konsistenz entstanden ist. 72,4 ml Wasser werden unter weiterem Rühren langsam zugesetzt, und man erhält eine klare, stabile Phospholipidlösung, die einen pH-Wert um den Neutralpunkt hat.

*Beispiel 9*

12 g N-(2-Hydroxyethyl)-heptansäureamid werden bei 20° C in 10 ml Wasser gelöst. Nach Zugabe von 16 g Phospholipid der Zusammensetzung aus Beispiel 7 wird solange bei 20° C gerührt, bis eine klare Lösung von öliger Konsistenz entstanden ist. 62 ml Wasser werden unter weiterem Rühren langsam zugesetzt, und man erhält eine klare, stabile Phospholipidlösung, die einen pH-Wert um den Neutralpunkt hat.

*Beispiel 10*

6 g N-(2-Hydroxyethyl)-valeriansäureamid werden bei 20° C in 10 ml Wasser gelöst. Nach Zugabe von 4 g Phospholipid der Zusammensetzung

| | |
|---|---|
| Phosphatidylcholin | 26,2% |
| Phosphatidylethanolamin | 25,1% |
| Lysophosphatidylcholin | 1,3% |
| Phosphatidylinosit | 21,4% |
| N-Acylphosphatidylethanolamin | 5,3% |
| Phosphatidsäure | 12,6% |
| Glycolipide und Triglyceride | 7,8% |

wird solange bei 20° C gerührt, bis eine klare Lösung von öliger Konsistenz entstanden ist. 80 ml Wasser werden unter weiterem Rühren langsam zugesetzt, und man erhält eine klare, stabile Phospholipidlösung, die einen pH-Wert um den Neutralpunkt hat.

*Beispiel 11*

9,6 g N-(2-Hydroxyethyl)-capronsäureamid werden bei 20° C in 10 ml Wasser gelöst. Nach Zugabe von 8 g Phospholipid der Zusammensetzung aus Beispiel 10 wird solange bei 20° C gerührt, bis eine klare Lösung von öliger Konsistenz entstanden ist. 72,4 ml Wasser werden unter weiterem Rühren langsam zugesetzt, und man erhält eine klare, stabile Phospholipidlösung, die einen pH-Wert um den Neutralpunkt hat.

*Beispiel 12*

14,4 g N-(2-Hydroxyethyl)-capronsäureamid werden bei 20° C in 10 ml Wasser gelöst. Nach Zugabe von 12 g Phospholipid der Zusammensetzung aus Beispiel 10 wird solange bei 20° C gerührt, bis eine klare Lösung von öliger Konsistenz entstanden ist. 63,6 ml Wasser werden unter weiterem Rühren langsam zugesetzt, und man erhält eine klare, stabile Phospholipidlösung, die einen pH-Wert um den Neutralpunkt hat.

## Patentansprüche

1. Wässerige Phospholipidlösungen, dadurch gekennzeichnet, dass sie als Lösungsvermittler Hydroxyethylamide der allgemeinen Formel I

$$CH_3(CH_2)_nCONH-CH_2-CH_2-OH \quad I$$

in der n = 3,4 oder 5 bedeutet, enthalten.

2. Wässerige Phospholipidlösungen gemäss Anspruch 1, gekennzeichnet durch den Gehalt von 1-25 Teilen Phospholipid und 0,75-37,5 Teilen eines Hydroxyethylamids der Formel I.

3. Wässerige Phospholipidlösung gemäss Ansprüchen 1-2, dadurch gekennzeichnet, dass sie als Hydroxyethylamid N-(2-Hydroxyethyl)-valeriansäureamid enthält.

4. Wässerige Phospholipidlösung gemäss Ansprüchen 1-2, dadurch gekennzeichnet, dass sie als Hydroxyethylamid N-(2-Hydroxyethyl)-capronsäureamid enthält.

5. Wässerige Phospholipidlösung gemäss Ansprüchen 1-2, dadurch gekennzeichnet, dass sie als Hydroxyethylamid N-(2-Hydroxyethyl)-heptansäureamid enthält.

6. Wässerige Phospholipidlösung gemäss Ansprüchen 1-5, dadurch gekennzeichnet, dass sie als Phospholipid Phosphatidylcholin enthält.

7. Verwendung von Hydroxyethylamiden der allgemeinen Formel I als Lösungsvermittler für Phospholipide.

8. Verwendung von Hydroxyethylamiden als Lösungsvermittler für Phospholipide gemäss Anspruch 7, dadurch gekennzeichnet, dass N-(2-Hydroxyethyl)-valeriansäureamid eingesetzt wird.

9. Verwendung von Hydroxyethylamiden als Lösungsvermittler für Phospholipide gemäss Anspruch 7, dadurch gekennzeichnet, dass N-(2-Hydroxyethyl)-capronsäureamid eingesetzt wird.

10. Verwendung von Hydroxyethylamiden als Lösungsvermittler für Phospholipide gemäss Anspruch 7, dadurch gekennzeichnet, dass N-(2-Hydroxyethyl)-heptansäureamid eingesetzt wird.

11. Verfahren zur Herstellung von wässerigen Phospholipidlösungen gemäss Ansprüchen 1-6, dadurch gekennzeichnet, dass man Phospholipide in Gegenwart eines Lösungsvermittlers der allgemeinen Formel I in Wasser löst.

## Claims

1. Aqueous solutions of phospholipids, characterized in that they contain as solubilizer hydroxyethyl amides of the general formula I

$$CH_3(CH_2)_nCONH-CH_2-CH_2-OH \quad I$$

wherein n is 3, 4 or 5.

2. Aqueous solutions of phospholipids according to Claim 1, characterized by a content of 1 to 25 parts of phospholipid and 0.75 to 37.5 parts of a hydroxyethyl amide of formula I.

3. Aqueous solution of phospholipids according to Claims 1 and 2, characterized in that it contains N-(2-hydroxyethyl) valerianic acid amide as hydroxyethyl amide.

4. Aqueous solution of phospholipids according to Claims 1 and 2, characterized in that it contains N-(2-hydroxyethyl) capronic acid amide as hydroxyethyl amide.

5. Aqueous solution of phospholipids according to Claims 1 and 2, characterized in that it contains N-(2-hydroxyethyl) heptanic acid amide as hydroxyethyl amide.

6. Aqueous solution of phospholipids according to Claims 1 to 5, characterized in that it contains phosphatidyl choline as phospholipid.

7. Use of hydroxyethyl amides of general formula I as solubilizer for phospholipids.

8. Use of hydroxyethyl amides as solubilizer for phospholipids according to Claim 7, characterized in that there is used N-(2-hydroxyethyl) valerianic acid amide.

9. Use of hydroxyethyl amides as solubilizer for phospholipids according to Claim 7, characterized in that there is used N-(2-hydroxyethyl) capronic acid amide.

10. Use of hydroxyethyl amides as solubilizer for phospholipids according to Claim 7, characterized in that there is used N-(2-hydroxyethyl) heptanic acid amide.

11. Process for the production of aqueous solutions of phospholipids according to Claims 1 to 6, characterized in that the phospholipids are dissolved in water in the presence of a solubilizer of general formula I.

## Revendications

1. Solutions aqueuses de phospholipides, caractérisées en ce qu'elles contiennent en tant que tiers-solvants des hydroxyéthylamides de formule générale I

$$CH_3(CH_2)_nCONH-CH_2-CH_2-OH \quad I$$

dans laquelle n = 3, 4 ou 5.

2. Solutions aqueuses de phospholipides selon la revendication 1, caractérisées en ce qu'elles contiennent de 1 à 25 parties de phospholipide et de 0,75 à 37,5 parties d'un hydroxyéthylamide de formule I.

3. Solution aqueuse de phospholipide selon les revendications 1 et 2, caractérisée en ce qu'elle contient en tant qu'hydroxyéthylamide le N-(2-hydroxyéthyl)-valéramide.

4. Solution aqueuse de phospholipide selon les revendications 1 et 2, caractérisée en ce qu'elle contient en tant qu'hydroxyéthylamide le N-(2-hydroxyéthyl)-caproamide.

5. Solution aqueuse de phospholipide selon les revendications 1 et 2, caractérisée en ce qu'elle contient en tant qu'hydroxyéthylamide le N-(2-hydroxyéthyl)-heptanamide.

6. Solution aqueuse de phospholipide selon les revendications 1 à 5, caractérisée en ce qu'elle contient en tant que phospholipide la phosphatidylcholine.

7. Utilisation des hydroxyéthylamides de formule générale I en tant que tiers-solvants pour des phospholipides.

8. Utilisation des hydroxyéthylamides en tant que tiers-solvants pour des phospholipides selon la revendication 7, caractérisée en ce que l'on utilise le N-(2-hydroxyéthyl)-valéramide.

9. Utilisation d'hydroxyéthylamides en tant que tiers-solvants pour des phospholipides selon la revendication 7, caractérisée en ce que l'on utilise le N-(2-hydroxyéthyl)-caproamide.

10. Utilisation d'hydroxyéthylamides en tant que tiers-solvants pour des phospholipides selon la revendication 7, caractérisée en ce que l'on utilise le N-(2-hydroxyéthyl)-heptanamide.

11. Procédé de préparation de solutions aqueuses de phospholipides selon les revendications 1 à 6, caractérisé en ce que l'on dissout les phospholipides dans l'eau en présence d'un tiers-solvant de formule générale I.